# EUROPEAN PATENT SPECIFICATION

(11) **EP 1 414 383 B1**
(45) Date of publication and mention of the grant of the patent: **26.07.2006**
(21) Application number: 02755129.0
(22) Date of filing: 06.08.2002
(51) Int. Cl.: A61H 3/00, A61D 9/00, A61F 5/01, A61B 17/00

(54) **WALKING AID**
GEHHILFE
AIDE A LA MARCHE

(30) Priority: 06.08.2001 GB 0119104
(43) Date of publication of application: 06.05.2004
(73) Proprietor: Patterson, Noble, Co Tyrone BT70 2EY (GB)
(72) Inventor: Patterson, Noble, Co Tyrone BT70 2EY (GB)
(74) Representative: Main, Malcolm Charles
(86) International application number: PCT/GB2002/003627
(87) International publication number: WO 2003/013415

(56) References cited:
- GB-A- 196 117
- GB-A- 657 446
- US-A- 1 660 721
- US-A- 4 099 525
- US-A- 5 230 700
- US-A- 5 578 041

## Description

The present invention concerns a walking aid that is attachable to leg frames used to help leg problems such as broken or fractured bones or leg deformities in human or animal legs to heal and be correct, by assisting "early walking".

GB 657,446 relates to a U-shaped leg or foot support, for use with plaster casts, having resilient side members and a bottom cross member. In use, the resilience of the side members means that the impact of the bottom cross member on the ground is transferred gradially to the injured leg.

Currently, when patients have traumatic leg injuries such as large or multiple breaks in the major bones of the lower leg, a frame, such as an Ilizarov frame or the American Taylor Spatal frame, is fixedly attached around and through the lower leg using pins that pierce the skin and relevant bones. This support provides a stable frame around the lower leg, and aligns the bone fragments correctly so that they may in time reattach to re-form into a single bone structure. Such lower leg frames also aid walking by the patient as soon as possible after the frame has been fixed to the injured leg.

One of the most important aspects of bone healing is that the bone cells (chondrocytes and osteocytes) involved in producing and developing new bone tissue, respond to pressure on the bone, e.g. by walking, to help them grow.

However, often one of the greatest obstacles for the patient to overcome is the mental block in using the framed leg to walk. The patient must also gauge how much pressure he or she can apply to the injured leg without causing excess pain and discomfort. Unfortunately, this often leads to ineffective use of the frame and possibly prolonged and unnecessarily extended recovery periods for the patient. This is particularly exacerbated when the patient has a broken or severely damaged ankle as well.

Thus, according to one aspect of the present invention there is provided A leg support comprising a human or animal leg frame, for use with broken or fractured bones, or leg deformities, which, in use, is fixedly attached around and through the leg using pins that pierce the skin and relevant bones, characterised in that said leg support further comprises a walking aid wherein the aid comprises at least one weight-bearing gas strut actuator adapted to take some or all of the user's weight when walking.

The walking aid, attached to the leg frame, is used by patients to help them walk, which in turn facilitates rapid recovery and re-growth of the leg bone by improving mobility. It also reduces dependency on wheelchairs, time and effort in hospital, and overall recovery time.

In addition, certain leg or other orthopaedic deformities or problems are currently improved or assisted with using similar leg frames. Early walking with these could also be provided by the present invention.

Preferably, the walking aid uses two or more actuators.

Preferably the actuator(s) are piston or ram and housing arrangements, such as gas struts, and they are permanently or separably fixable to the outside of the leg frame. The actuators are preferably positioned substantially symmetrically around the frame.

The actuator(s) are preferably dampened and/or have another controlled extension/retraction action.

Preferably the gas strut is a hydraulic gas strut.

Preferably the walking aid is adjustable to accommodate the weight of differently sized patients. The weight bearing capacity of the walking aid could also be adjusted to get the desired amount of pressure on the injured foot or ankle by applying the required balance of upward pressure to the frame.

The walking aid preferably further include flexible fittings and/or mountings such as rubber mountings, rose joints, intermediate swivel means, as well as swivel bases, possibly involving universal joint means, to assist or allow twisting motion of the leg that normally occurs during walking.

Between the patient's foot and the walking aid, there may also be a connecting sleeve that connects onto the foot and the base of the walking aid. Such a sleeve could be made in a range of lengths so as to get correct strut lengths for different frame configurations.

The walking aid is attachable to any suitable leg frame, including an Ilizarov frame and an American Taylor Spatial frame.

An embodiment of the present invention will herein be described by way of example only and with reference to the accompanying drawings in which;
Figure 1 as a diagrammatic front representation of a walking aid attached to an Ilizarov frame fixed to a human leg; and

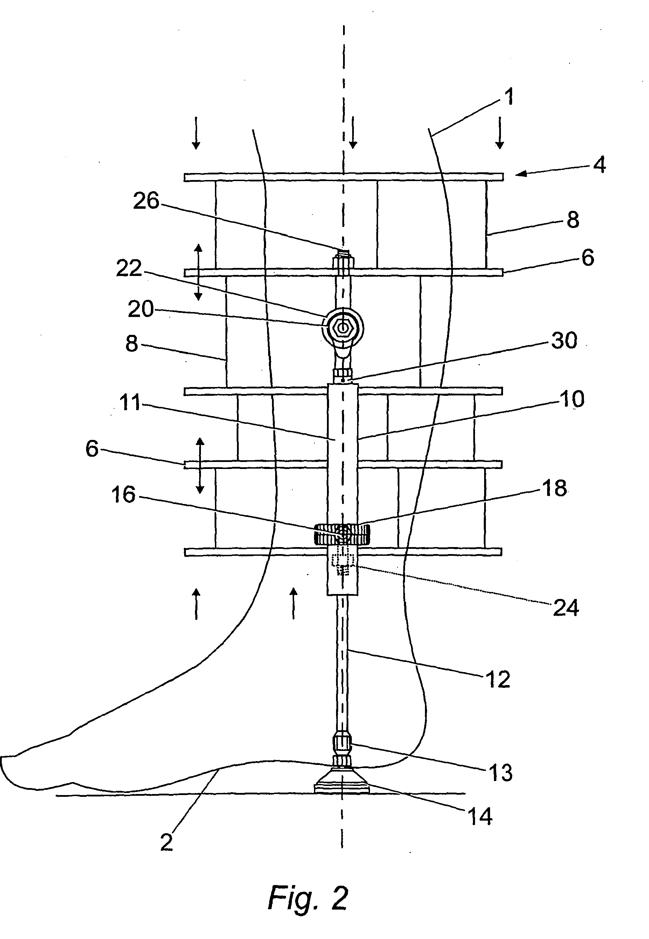

Figure 2 is a profile view of a Figure 1.

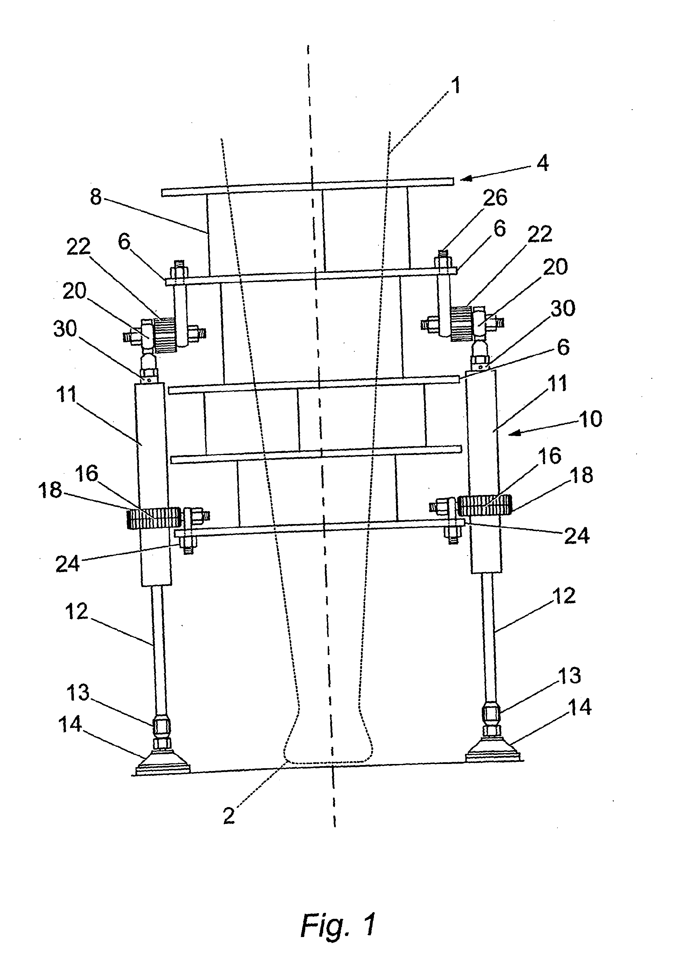

Figure 1 shows a (diagrammatic) human lower leg 1 and human foot 2. A (diagrammatic) Ilizarov frame 4 is shown composed of a number of carbon fibre rings 6 that are interconnected via vertical bars 8. The frame is fixed to the human lower leg 1 via pins (not shown) that extend across the inner circumference of the carbon fibre rings 6, through the leg and the bone that is fractured or broken.

The walking aid is composed of two adjustable stainless steel gas struts 10 which each have a stainless steel housing 11, a resistibly urged piston 12, a height adjusting sleeve 13, and a stainless steel foot with a swivel base 14. The upper part of the gas strut 10 is attached to an upper carbon fibre ring 6 via outriggers at points 26. On each strut 10 is an alloy swivel 18 attached via outriggers at points 24 to a lower frame ring 6, which allow height adjustment relative to the lowest frame ring 6.

At the top of each strut 10 there is a stainless steel rose joint 20 and a rubber mount 22, which act together to allow twisting motion of the knee joint during the motion of walking.

A grub screw 30 is situated at the top of each stainless steel gas strut 10, and allows controlled release of gas from the gas strut 10.

In operation, the Ilizarov frame 4 is fixed to the lower leg 1 of the patient via pins that extend across the inner circumference of the carbon fibre rings 6 into and through skin of the leg 1 and the bone fragments that need to re-grow and re-form.

An example of steps to be followed for fitting and adjusting the struts 10 is set out below.
- Weigh patient.
- Get patient to push down on scales with their damaged foot to determine what weight their limb can take comfortably.
- Subtract that weight from their body weight.
- Divide remaining weight between the two gas struts i.e. 17 stone body weight, 6 stone on damaged limb, 11 stone remaining = 5½ stone on each strut.
- To preset struts - push on scales with gas strut and release gas from grub screw 30 until required weight is obtained.
- With patient standing hold the gas strut beside the frame to determine appropriate mounting position on frame. You may have to fit shorter or longer height adjusting sleeve 13 as needed.
- Mount outrigger 26 to top position (outrigger with slot).
- Mount rubber mounting 22 to outrigger 26 putting male side through and fitting nut.
- Fit two-hole outrigger 24 to appropriate position at bottom of frame.
- Fit alloy swivel 18 to outrigger 24.
- Slide gas strut through swivel 18 and connect top to rubber mount 22 with 6mm bolt supplied.
- With patient standing, adjust struts so that strut will compress when weight is put on damaged limb.

The patient can now stand by applying weight to the injured leg 1. Some if not the majority of the patient's weight normally applied through the injured leg 1 will be born by the walking aid struts 10. when the patient stands, the stainless steel feet with swivel base 14 come in contact with the floor and bear the desired pressure according to the gas pressure set in each strut 10.

While the patient takes an initial step and lifts the injured leg 1 from the ground, the pressure is removed from the gas struts 10 so that the pistons 12 are in their generally extended position. When the patient finishes the step by again applying pressure on the foot 2 of the injured leg 1, the resistibly urged pistons 12 are pushed into their contracted position. The normal twisting action of the knee joint that occurs when the foot of a healthy individual hits the floor is allowed via the twisting motion of the rubber mounts 22. This swivel motion is further accommodated via the alloy swivels 18. The extension and contraction of the pistons 12 are controlled by hydraulics within the stainless steel housing 11.

Thus, by a simple adjustment of the gas pressure within the struts of the walking aid attached to a deformed, fractured- or broken-leg frame, the patient can feel confident to apply pressure on the injured leg, as the walking aid will bear the precise amount of weight and pressure that the patient feels comfortable with. The patient can therefore walk without fearing excessive pain due to accidental over loading of the injured leg while walking or standing, which will significantly increase the rate of recovery and re-growth of the fractured or broken bone and so reduce the use of crutches, wheelchairs etc. and minimise the need for physiotherapy and other forms of recovery treatments.

## Claims

1. A leg support comprising a human or animal leg frame (4), for use with broken or fractured bones, or leg deformities, which, in use, is fixedly attached around and through the leg using pins that pierce the skin and relevant bones, **characterised in that** said leg support further comprises a walking aid wherein the aid comprises at least one weight-bearing gas strut actuator (10) adapted to take some or all of the user's weight when walking.

2. The leg support as claimed in claim 1 wherein the gas pressure within said weight-bearing gas strut (10) is adjustable.

3. The leg support as claimed in claim 1 or 2 comprising at least two actuators (10).

4. The leg support as claimed in any preceding claim wherein the actuator(s) (10) are fixed to the outside of the leg frame.

5. The leg support as claimed in any of claims 3 or 4 wherein the actuator(s) are positioned substantially symmetrically around the frame.

6. The leg support as claimed in any one of the preceding claims wherein the actuator(s) (10) are dampened and/or have another controlled extension/retraction action.

7. The leg support as claimed in any one of the preceding claims wherein the gas strut (10) is a hydraulic gas strut.

8. The leg support as claimed in any one of the preceding claims wherein the aid comprises at least one fitting (20, 22) chosen from the group consisting of flexible fittings and mountings such as rubber mountings, rose joints, intermediate swivel means, as well as swivel bases, involving universal joint means, to assist or allow twisting motion of the leg that normally occurs during walking.

9. The leg support as claimed in any one of the preceding claims which further comprises a connecting sleeve that is connectable between a foot and the base of the walking aid.

10. The leg support as claimed in any one of the preceding claims wherein the frame (4) to which the walking aid is attached is an Ilizarov frame or an American Taylor Spatial frame.

11. The leg support as claimed in any one of the preceding claims wherein, in use, the frame (4) is fixedly attached around and through the lower leg.

## Patentansprüche

1. Eine Beinstütze, die einen Menschen- oder Tierbeinrahmen (4) beinhaltet, zur Verwendung mit gebrochenen oder frakturierten Knochen oder Beindeformitäten, die bei der Verwendung unbeweglich um und durch das Bein unter Verwendung von Stiften, die die Haut und relevanten Knochen durchbohren, angebracht wird, **dadurch gekennzeichnet, dass** die Beinstütze ferner eine Gehhilfe beinhaltet, wobei die Hilfe zumindest einen Gewicht tragenden Gasstreben-Betätigungszylinder (10) beinhaltet, der ausgeführt ist, um ein wenig des Gewichts oder das gesamte Gewicht des Benutzers beim Gehen zu übernehmen.

2. Beinstütze gemäß Anspruch 1, wobei der Gasdruck innerhalb der Gewicht tragenden Gasstrebe (10) einstellbar ist.

3. Beinstütze gemäß Anspruch 1 oder 2, die zumindest zwei Betätigungszylinder (10) beinhaltet.

4. Beinstütze gemäß einem der vorhergehenden Ansprüche, wobei der/die Betätigungszylinder (10) an der Außenseite des **Beinrahmens befestigt ist/sind.**

5. Beinstütze gemäß Anspruch 3 oder 4, wobei der/die Betätigungszylinder im Wesentlichen symmetrisch um den Rahmen herum positioniert ist/sind.

6. Beinstütze gemäß einem der vorhergehenden Ansprüche, wobei der/die Betätigungszylinder (10) gedämpft ist/sind und/oder eine andere gesteuerte Ausfahr-/Einziehtätigkeit aufweist/aufweisen.

7. Beinstütze gemäß einem der vorhergehenden Ansprüche, wobei die Gasstrebe (10) eine Hydraulikgasstrebe ist.

8. Beinstütze gemäß einem der vorhergehenden Ansprüche, wobei die Hilfe zumindest ein Anschlussstück (20, 22) beinhaltet, das aus der Gruppe, bestehend aus biegsamen Anschlussstücken und Halterungen, wie etwa Gummihalterungen, Gelenkköpfen, Zwischenschwenkmittel sowie Schwenkbasen, mit Universalgelenkmitteln ausgewählt ist, um bei der normalerweise während des Gehens vorkommenden Verdrehbewegung des Beines behilflich zu sein oder diese zu ermöglichen.

9. Beinstütze gemäß einem der vorhergehenden Ansprüche, die ferner eine Anschlussmuffe beinhaltet, die zwischen einem Fuß und der Basis der Gehhilfe angeschlossen werden kann.

10. Beinstütze gemäß einem der vorhergehenden Ansprüche, wobei der Rahmen (4), an dem die Gehhilfe angebracht ist, ein llizarov-Frame oder ein amerikanischer Taylor-Spatial-Frame ist.

11. Beinstütze gemäß einem der vorhergehenden Ansprüche, wobei der Rahmen (4) bei der Verwendung unbeweglich um und durch den Unterschenkel angebracht ist.

## Revendications

1. Un support pour jambe comportant un cadre (4) pour jambe humaine ou animale, destiné à être utilisé avec des os brisés ou fracturés, ou des difformités de jambe, lequel, lors de l'utilisation, est attaché de façon fixe autour et au travers de la jambe à l'aide de broches qui percent la peau et les os appropriés, **caractérisé en ce que** ledit support pour jambe comporte de plus une aide à la marche dans lequel l'aide comporte au moins un actionneur d'étai à gaz portant (10) adapté pour supporter une partie ou la totalité du poids de l'utilisateur lors de la marche de celui-ci.

2. Le support pour jambe tel que revendiqué dans la revendication 1 dans lequel la pression de gaz au sein dudit étai à gaz portant (10) est réglable.

3. Le support pour jambe tel que revendiqué dans la revendication 1 ou la revendication 2 comportant au moins deux actionneurs (10).

4. Le support pour jambe tel que revendiqué dans n'importe quelle revendication précédente dans lequel le ou les actionneurs (10) sont fixés sur l'extérieur du cadre pour jambe.

5. Le support pour jambe tel que revendiqué dans n'importe lesquelles des revendications 3 ou 4 dans lequel le ou les actionneurs sont positionnés de façon substantiellement symétrique autour du cadre.

6. Le support pour jambe tel que revendiqué dans n'importe laquelle des revendications précédentes dans lequel le ou les actionneurs (10) sont amortis et/ou possèdent une autre action commandée d'extension/de rétraction.

7. Le support pour jambe tel que revendiqué dans n'importe laquelle des revendications précédentes dans lequel l'étai à gaz (10) est un étai à gaz hydraulique.

8. Le support pour jambe tel que revendiqué dans n'importe laquelle des revendications précédentes dans lequel l'aide comporte au moins une garniture (20, 22) choisie dans le groupe consistant en garnitures et montures flexibles telles que des montures en caoutchouc, des joints à rotule, des moyens pivotants intermédiaires, ainsi que des bases pivotantes, dont des moyens formant joints universels, pour aider ou permettre le mouvement de torsion de la jambe qui se produit normalement durant la marche.

9. Le support pour jambe tel que revendiqué dans n'importe laquelle des revendications précédentes qui comprend de plus un manchon de raccord qui peut être raccordé entre un pied et la base de l'aide à la marche.

10. Le support pour jambe tel que revendiqué dans n'importe laquelle des revendications précédentes dans lequel le cadre (4) auquel est attaché l'aide à la marche est un cadre Ilizarov ou un cadre Taylor Spatial américain.

11. Le support pour jambe tel que revendiqué dans n'importe laquelle des revendications précédentes dans lequel, lors de l'utilisation, le cadre (4) est attaché de façon fixe autour et au travers de la jambe inférieure.
